# EUROPEAN PATENT SPECIFICATION

(11) **EP 0 638 083 B1**
(45) Date of publication and mention of the grant of the patent: **12.03.1997**
(21) Application number: 93911469.0
(22) Date of filing: 16.04.1993
(51) Int. Cl.: C07F 9/10, A61K 31/685, A61K 35/30

(54) **METHOD FOR THE PREPARATION AND PURIFICATION OF PHOSPHOLIPID MIXTURES FREE FROM CONTAMINATION BY UNCONVENTIONAL VIRUSES**
VERFAHREN ZUR HERSTELLUNG UND REINIGUNG VON PHOSPHOLIPIDMISCHUNGEN FREI VON KONTAMINIERENDEN UNKONVENTIONELLEN VIREN
PROCEDE DE PREPARATION ET DE PURIFICATION DE MELANGES DE PHOSPHOLIPIDES NON CONTAMINES PAR DES VIRUS NON CLASSIQUES

(30) Priority: 17.04.1992 IT PD920007
(43) Date of publication of application: 15.02.1995
(73) Proprietor: FIDIA S.p.A., I-35031 Abano Terme (Padova) (IT)
(72) Inventor: DI MARTINO, Alessandro, I-35036 Montegrotto Terme (PD) (IT); CALLEGARO, Lanfranco, I-35020 Ponte di Brenta (PD) (IT); TOFFANO, Gino, I-35036 Montegrotto Terme (PD) (IT)
(74) Representative: VOSSIUS & PARTNER
(86) International application number: EP9300915
(87) International publication number: WO9321190

(56) References cited:
- EP-A- 0 148 045
- EP-A- 0 150 712
- WO-A-91/07417
- CHEMICAL ABSTRACTS, vol. 101, no. 22, 26 November 1984, Columbus, Ohio, US, abstract no. 198172c, p. 377

## Description

The present invention relates to a process for the preparation of phospholipid mixtures which selectively eliminates contaminants associated with potentially pathogenic unconventional viruses, without altering the biological and pharmacological characteristics of the mixture.

Extracts from bovine brain have been used as therapeutic agents for many years. Of such extracts, purified phospholipids represent the active principle of a large number of pharmacological specialties. Experimental and clinical documentation of the pharmacological properties of phospholipids have been available for many years (Bruni A. et al., Nature 260, 331, 1976; Hirata F. et al., Nature 275, 219, 1978; Delwaide P.J. et al., Acta Neurol. Scand. 73, 136, 1986).

It has long been known that it is possible to extract, at research level, mixtures of phospholipids (which have not yet been fully characterized) (J. Folch, et al., J. Biol. Chem. 226: 497, 1957; N. Radin, Methods Enzymol. 72: 5, 1981; E.G. Bligh, Can. J. Biochem. Physiol. 37: 911, 1959), but none of the aforesaid methods was developed with a view to demonstrating the elimination and destruction of components associated with unconventional viruses. One reason therefor is that at the time the above mentioned procedure was found such diseases caused by unconventional viruses were not known to affect the mammalian species whose brains were used for extraction. Another reason is that no available method for the identification of potentially dangerous components was available. Pathological conditions may sometimes occur where the pathogenic agent or agents have not been identified. One such pathology, called bovine spongiform encephalopathy (BSE), was first reported in England in 1986 (Wells G. et al. Vet. Record, 419, 1987).

The disease is so called because of the spongy appearance of the brain tissue of affected animals when observed under a microscope; the main lesions are constituted by extensive intraneuronal vacuolation.

All current evidence indicates that BSE belongs to the group of degenerative encephalopathies of the central nervous system, caused by the family of unconventional, transmissible agents whose outcome is invariably fatal (Fraser et al., Vet. Record 123: 472, 1988; Hope et al., Nature 336: 390, 1988). Such diseases include scrapie of sheep and goats, the chronic wasting disease (CWD) which affects captive mule deer, transmissible mink encephalopathy affecting animals on mink ranches, and three human diseases: kuru, Creutzfeldt-Jakob disease (CJD) and the Gerstmann-Straüssler-Scheinker syndrome (GSS). The histopathological lesions found in brains affected by these diseases are similar to those caused by BSE.

Many theories have been put forward as to the nature of these etiological agents, which are different from any known infectious agent, and therefore known as unconventional agents. Due to the long incubation period between infection and the onset of clinical symptoms, they are also known as "slow viruses".

Since the first few cases in 1986, the disease has reached epidemic proportions in Britain, affecting over 40,000 head of cattle. Affected animals show no signs of the disease for several years (the incubation time is 4-5 years), but once the symptoms have appeared, the course of the disease is rapid and terminates invariably in death.

The results of an epidemiological study carried out by the Central Veterinary Laboratory of the British Ministry of Agriculture (Wilesmith et al, Vet. Record, 123: 638, 1988) identified the source of infection in concentrated animal foodstuffs made from the carcasses of ruminants and sold as meat and bone meal. As the encephalopathies can be transmitted to a wide range of animal species, it is feasible that BSE is the result of infection by the etiologic agent responsible for scrapie, transmitted to cattle in contaminated foodstuffs (Morgan KL, Vet. Record 122: 445, 1988).

On the basis of the results of these studies, the British government issued an order which came into effect on 18th July 1988, outlawing the sale and supply of animal foodstuffs containing proteins derived from ruminants.

It is commonly thought that various circumstances contributed to the sudden outbreak of BSE in the United Kingdom (Cherfas J., Science Feb. 1990, 523).

Firstly, the number of sheep in the U.K. increased rapidly in the late 70's and early 80's, and with it the occurrence of scrapie, an endemic disease of the ovine species in Europe for over 250 years (Pattison et al, Vet. Record 90: 465, 1972). At the same time, in the wake of the oil crisis, animal feed production plants switched their methods of production to a low temperature system which was probably less effective in destroying the highly resistant scrapie agent. All meat and bone meal producers except one discontinued the use of organic solvents, such as benzene, hexane and trichloroethylene, to remove excess fat from soybean and bone meal. Perhaps most significant of all was that the final stage of heating the products to remove the solvents was consequently left out: indeed this step required very high temperatures.

Moreover, government policy encouraged breeders to produce more milk, and wean calves early by feeding them protein-enriched diets.

These were often of poor quality, since protein meal made from meat and bone was cheaper than products made with soybean and fish which are more reliable sources of protein.

Studies on how BSE is transmitted are fundamental to research. The most important aspect of these experiments is that, by identifying the limits of the inter-species barriers to transmission of the pathogenic agent, it may be possible to assess the risk of infection by BSE to any one species. Fraser et al (Vet. Record, 123:472, 1988) demonstrated that the disease could be transmitted to mice by inoculating extracts from the brains of cattle which had died from BSE into the brains of mice which subsequently developed the disease. Later, Barlow et al (Vet. Record, 3 Feb. 1990) transmitted the disease to mice by feeding them infected brains. It was the first evidence that BSE could be transmitted by the oral route. No other tissue from infected animals (spleen, spinal cord, lymphatic tissues, milk etc.) has been able to produce the disease in mice. While evidence exists that scrapie can be transmitted from ewe to lamb, there is no proof so far of possible vertical or horizontal transmission of the etiological agent of BSE among cattle.

The agents which cause subacute spongiform encephalopathies are extremely resistant to standard decontamination procedures.

In D.M. Asher et al., Slow Viral Infections, in Laboratory Safety: principles and practices (ed. by Miller), 1986, p. 59-67, American Society Microb., it is reported that unconventional slow viruses can be inactivated by heat, sodium hydroxide or sodium hypochlorite. Currently available data on this aspect mainly originate from studies on the inactivation of scrapie and Creutzfeldt-Jakob disease agents.

The etiological agent of scrapie is highly resistant to temperature sterilization. Prolonged exposure to temperatures of up to 80°C only slightly reduces their infectivity; higher temperatures however markedly reduce infectivity (Hunter et al, J. Gen. Microbiol. 37: 251,1964). A small quantity of infectious "virus" remains viable when suspensions of infected material are heated to 100°C for 1 hour or to 118°C for 10 minutes. Recently the need was felt to update standards of sterilizing these infectious agents by steam autoclaving. Current methods of autoclaving in the United States for decontamination from Creutzfeldt-Jakob disease involve treatment at 132°C for 1 hour (Rosenberg et al, Annals of Neurology 19: 75, 1986), and is based on studies carried out on brain homogenates containing scrapie or Creutzfeldt-Jakob agents (Brown et al, J. of infectious diseases 153: 1145, 1986). In the U.K., current standards of autoclaving for decontamination from Creutzfeldt-Jakob disease involve treatment in an autoclave at 134-138°C for 18 minutes, and are based on various studies including one by Kimberlin (Kimberlin et al, Journal of Neurological Sciences 59: 355, 1983). Unfortunately, the spongiform encephalopathy agents are very resistant even to common chemical and physical treatments. Solvents such as benzene, hexane, petrol and trichloroethylene have been used as extraction solvents, but little is known of their effects on infectivity. Limited data are available on the chemical inactivation of the infectious agents, mainly because studies require large numbers of animals and long monitoring times.

Concentrations of 0.3% - 2.5% of sodium hypochlorite greatly reduced infectivity in the biological assays, but did not always eliminate it completely (Walker et al., Am. J. Publ. Health 73:661, 1983). Data regarding treatment with up to 0.25 N sodium hydroxide are inconsistent; however at a concentration of over 1 N it appears to be the most efficacious chemical agent of all those studied. Treatment with 6M-8M urea was also reported to be highly variable.

The results of the studies on decontamination thus show that, although most of the infectivity is quickly destroyed by many of the different physical and chemical treatments, the existence of small subpopulations of infectious agents resistant to inactivation makes complete sterilization of contaminated materials extremely difficult in practice.

Once BSE had been identified as a "scrapie-like" disease, important epidemiological and analytical questions were raised, the latter in particular being aimed at identifying the agent that could be associated with the infection.

The sudden appearance of BSE and all the other aspects of these neurological disorders still to be clarified have caused necessary consideration to be given to the problem, especially by those involved in the preparation of products deriving from bovine material.

Before BSE ever occurred it was obvious that the state of the art required a product to be pharmaceutically acceptable and free from those biological contaminants which were known at the time to constitute a potential risk to health. But clearly, the subsequent appearance of the above-described pathology in adult cattle has made it necessary to obtain an active principle which, without losing its known therapeutic properties, is characterized by the complete absence of unconventional viral agents, to be achieved by the use of specific procedures to guarantee the inactivation of these unconventional viral agents and the complete elimination of infectivity, and to use specific methodologies by which to identify such agents.

Indeed, it may not be enough to use raw material which has been certified as suitable for consumption, to obtain compounds or mixtures of the same for pharmaceutical purposes. Obviously, the elimination of infectivity, in this case BSE, must be assessed by analyzing its biological action *in vivo.* This would serve as a test of the various steps of the process, but not as an overall check of the same. The elimination of infectivity associable with bovine spongiform encephalopathy is preferably assessed by means of experiments involving spiking with infective agents. It is especially preferred that the analysis of destruction of the infective agent introduced by spiking is performed *in vivo.* This analysis of the biological action *in vivo* is necessary since scientists do not agree over associating the infection with certain proteins or their fragments attributable to the unconventional infective agents such as the proteins PrP^{SC} or PrP₂₇₋₃₀ associated with BSE. Clearly, the extraction process which eliminates infectivity must at the same time leave the biological activity of the active principle intact, since this is essential for its therapeutic use. (Committee for Proprietary Medicinal product. Ad hoc working party on biotechnology-pharmacy; Biologicals 19: 247, 1991). Scientific research has produced, on the one hand, methods which guarantee suitable mixtures of phospholipids or their single fractions to be obtained devoid of protein, chemical and biological contaminants. On the other hand, methods with demonstrated efficacy in destroying infectivity associated with slow viruses are known, but no method exists by which it is possible to obtain, on an industrial scale, a product, as desired, in pure, pharmacologically active form, free from infectivity associable with pathogenic agents definable as slow viruses.

The purpose of the present invention is to provide a product in pharmacologically active form and, at the same time, being characterized by the absence of slow viruses. Such a product can be obtained by an advantageous process which can be applied to industrial production. The process offers innovative qualities founded on the suitable sequencing of its various extraction steps. During the various steps of this process, infective contaminants associable with slow viruses such as the bovine spongiform encephalopathy agent are eliminated, while the activity of the mixture, which represents the therapeutic activity of the product itself, remains unaltered. The product deriving from this process is constituted by a definite mixture of phospholipids or single fractions obtained from bovine brain or parts of the same.

According to the present invention, a phospholipid mixture which is essentially free from infective components while maintaining the pharmacological properties of said phospholipid mixture is prepared by a process comprising subjecting a material derived from nervous tissue a purification step selected from the group consisting of
A) extracting said material with an organic solvent,
B) loading a suspension of said material on a silica gel column and eluting said column with a mixture of organic and aqueous solvents, and a combination of steps A) and B).

The nervous tissue is preferably derived from bovine brain.

In a preferred variant of step A) said nervous tissue material is subjected to an extraction with a solvent mixture including a chlorinated hydrocarbon to obtain a first raw phospholipid-comprising mixture, subjecting said first raw phospholipid-comprising mixture a further purification step, and isolating the further purified product.

According to the invention, it is preferred that the suspension of the phospholipid mixture to be loaded on a silica gel column has been prepared by a method comprising the steps of
a) extracting bovine brain to obtain a crude extract comprising phospholipids,
b) filtering said crude extract to obtain a filtrate,
c) adding a precipitating agent to said filtrate to obtain a precipitate,
d) isolating said precipitate to obtain said first raw phospholipid-comprising mixture,
e) purifying said first raw mixture to obtain a second phospholipid-comprising mixture,
f) suspending said second mixture in a liquid.

The extraction in step a) is conducted with a mixture of organic solvents such as acetone in combination with chlorinated hydrocarbons, especially 1,1,1-trichloroethane. The extraction is performed under vigorous stirring for an extended period of time, e.g. 30-60 minutes. The temperature is preferably room temperature. After the extraction process which results in a suspension, the suspension is centrifuged, whereby insoluble material and the upper acetone-comprising phase were discarded. The crude extract is then filtered according to step b). In step c) a precipitating agent such as acetone is added to the filtrate; the mixture is stirred for about 30-60 minutes at a temperature below room temperature, e.g. at 13-17°C, preferably at 15°C. Thereafter the precipitate is isolated in step d) by means of centrifugation. The isolated product is the first raw material (phospholipid-comprising mixture). Said first raw material is redissolved in a solvent mixture comprising a chlorinated hydrocarbon such as 1,1,1-trichloroethane in admixture with an alcohol such as absolute ethanol. Thereby the first raw material is solubilized. The purification in step e) of the first raw material is performed by the addition of water, shaking the mixture for a period of 30-90 minutes, preferably 60 minutes, at room temperature. Separation of the phases is then performed by centrifugation. The process of solubilization and precipitation and centrifugation may be repeated. When the organic phase containing the phospholipid material has been isolated it is precipitated by means of e.g. acetone, and the mixture is left standing for 30-60 minutes at a temperature of 13-17°C, preferably 15°C. The precipitate is isolated by centrifugation and may be further rinsed with acetone. The precipitate is dried, e.g. in vacuum, to yield a second raw material (a second phospholipid-comprising mixture). This second raw material may be further purified by the addition of a saline to a suspension of the second raw material in a chlorinated hydrocarbon such as chloroform in admixture with an alcohol such as methanol. The mixture of aqueous and non-aqueous solvents comprising the second raw material is then shaken for 15-30 minutes at room temperature. Centrifugation of the two phases and recovery of the organic phase yields a solution of the desired phospholipid mixture. The phospholipids can be precipitated by means of acetone; in order to ensure full precipitation the mixture may be left standing for a period of time of 30-60 minutes at a temperature below room temperature, e.g. at 13-17°C, preferably 15°C. Centrifugation, rinsing with acetone and drying yield the final product. The final product may be sterilized by being exposed to elevated temperature.

The alternative purification method for purification consists of loading the material derived from nervous tissue on a silica gel column. The column is pre-washed with a chlorinated hydrocarbon such as chloroform before the nervous tissue derived material is loaded thereon. The elution of the desired phospholipid mixture is performed with the chlorinated hydrocarbon, preferably chloroform, to which is added one or more alcohols and water. The alcohol is selected from the group consisting of methanol, ethanol, propanols, butanols and pentanols. The elution is performed with eluents in e.g. the following sequence: chloroform, chloroform/ethanol, chloroform/methanol/water (4%) and chloroform/methanol/water (12.5%). The flow is divided in fractions which are analyzed for the content of phospholipids. Relevant fractions are pooled, and the phospholipids are recovered and dried.

When a combination of extraction and column chromatography is used in the purification process according to the invention, the second raw material from step e) is suspended in a suitable liquid comprising a chlorinated hydrocarbon such as chloroform and an alcohol such as methanol. The suspension is loaded on a silica gel column and eluted as described above. After the elution the desired end product is recovered from the eluting solvents, preferably by means of vacuum drying.

An especially preferred process variant consists of extraction of phospholipids from bovine brain by a mixture of chlorinated solvents, for example 1,1,1-trichloroethane, and acetone, at room temperature and for at least 30 minutes; or silica gel chromatography of a phospholipid mixture with elution with a succession of suitable mixtures of chloroform, ethanol, methanol and water; or a combination of the above procedures.

In a preferred variant of the process according to the invention the product is a phospholipid mixture comprising at least two components selected from the group consisting of phosphatidylcholine, phosphatidylethanolamine, phosphatidylserine, plasmalogen choline, plasmalogen ethanolamine, plasmalogen serine, phosphatidic acid, diphosphoinositide and sphingomyelin. In still another variant, the phospholipid preparable by the invention comprises only a single phospholipid fraction such as phosphatidylserine.

### MATERIALS AND METHODS

The bovine brains used in the process of extraction of the phospholipid mixture showed, on histological analysis, the fibrils typical for tissues belonging to materials from animals with the infection.

### BIOLOGICAL TEST FOR INFECTION

The animals used in these experiments were Golden Syrian hamsters (LVG/Lak). Tests for infection were carried out on groups of four female weanlings which had received intracerebral (i.c.) inoculation with 0.05 ml of the samples diluted ten times in sterile PBS. The intracerebral inoculations were effected by trained staff using disposable glass syringes with 26G, 3/8-inch sterile needles.

The final, sterilized product, concentrated 10 times, was used entirely.

The animals were examined twice a week or more for a period of 12 months, for signs of the characteristic, clinical, neurological symptoms. The onset of early symptoms in each animal was recorded, and the animals were sacrificed when the disease was in its final stage. Their brains were cut into halves, one fixed in 10% formalin and the other stored at -70°C. Histopathological examination was made in all animals which died of suspect causes and those which had shown signs of neurological disorders. At the end of the observation time, all surviving animals were sacrificed and a histopathological assessment was made on their brains.

The infective titer was calculated at the "final end point" according to the method of Reed and Munch, and is expressed as log LD₅₀/ml.

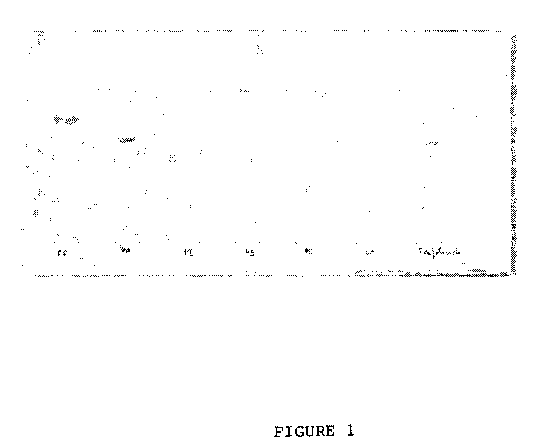

### Brief description of Figure 1

Figure 1 illustrates an example of the compositions of the phospholipid mixture purified as described.

### FIGURE 1

Example of silica gel chromatography of the following samples:
- Lane 1:: standard phospholipid phosphatidylethanolamine (PE)
- Lane 2:: standard phospholipid phosphatidic acid (PA)
- Lane 3:: standard phospholipid phosphatidylinositol (PI)
- Lane 4:: standard phospholipid phosphatidylserine (PS)
- Lane 5:: standard phospholipid phosphatidylcholine (PC)
- Lane 6:: standard phospholipid sphingomyelin (SM)
- Lane 7:: final product (phospholipid mixture)

The phospholipid mixture, obtained as described and free from contaminants associated with unconventional, potentially dangerous infective agents, can also be used for the preparation of single components of the phospholipid mixture, such as phosphatidylserine.

Thanks to its known pharmacological properties (G. Toffano et al., Pharmacol. Res. Comm. 8: 581, 1976; Bruni A. et al. Adv. Exp. Med. Biol. 72, 271-287, 1976; A. Leon et al., J. Neurochem. 30: 23, 1978; E. Boarato et al., Agents and actions 14: 613, 1984; Ponzin D. et al.: Immunopharm. 18, 167, 1989; Monastra G. et al. Lymphokine and cytokine Res. 11, 1, 39, 1992), the phospholipid mixture (or its single components), prepared by the process of the invention, can be used in general to prepare pharmaceutical compositions which are efficacious in numerous pathologies (with different etiopathogenic causes) in particular of the central nervous system and the immune system, especially degenerative pathologies associated with immune dysfunctions. The following can be cited: psychoorganic syndromes characterized by involution or cerebrovascular insufficiencies, behavioral impairments correlated with neuroendocrine alterations, depressive syndromes, anxious-depressive states, degenerative pathologies also associated with immune dysfunctions, multiple sclerosis, Alzheimer's disease, amyotrophic lateral sclerosis, encephalitis of various origin, and in pathologies associated with alterations in intestinal absorption of the fatty acids.

The pharmaceutical compositions can be administered by oral or parenteral route, preferably by intramuscular route, by intravenous injection or by infusion. The preparations can be as solutions of the compound (or freeze-dried powders of the compound) in association with one or more pharmaceutically acceptable vectors or diluents and contained in buffered media with a suitable pH and isotonic with the physiological fluids. Safe storage of the pharmaceutical can be ensured by packing it in vials, ampoules, capsules, single dose packets, as described in the following examples of pharmaceutical preparations. For its therapeutic and possibly also preventive application by said parenteral or oral routes, the dose of active substance depends on the desired effects and on the chosen route of administration and can be between 0.05 and 12 mg/kg body weight of the patient per dose, corresponding to a daily administration of between about 5 and 2000 mg to an adult human.

A method for the treatment of pathologies of the central nervous system and degenerative pathologies also associated with immune system dysfunctions comprises the administration of an effective amount of a phospholipid mixture prepared by the present invented process, to a patient in need therefor.

A mixture of phospholipids prepared according to the invention may have the following composition: 30-50% phosphatidylcholine, 24-44% phosphatidylethanolamine, 7-13% phosphatidylserine, and 11-27% phosphatidylinositol and minor phospholipids.

When the phospholipids are presented in pharmaceutical preparations, such preparations may have the following composition (expressed in mcg of phosphorus, per 50 mg of mixture): 280-420 mcg P as phosphatidylcholine, 140-210 mcg P as phosphatidylethanolamine, 140-210 mcg P as phosphatidylserine, 8-12 mcg P as plasmalogen choline, 68-102 mcg P as plasmalogen ethanolamine, 40-60 mcg P as plasmalogen serine, 10-24 mcg P as phosphatidic acid, 28-42 mcg P as diphosphoinositide and 68-102 mcg P as sphingomyelin.

Another composition of a mixture of phospholipids prepared according to the invention can be as follows (expressed in mcg of phosphorous per 100 mcg of total phosphorous): 25-45 mcg phosphatidylcholine, 12-22 mcg P as phosphatidylethanolamine, 12-22 mcg P as phosphatidylserine, 0.5-1.5 mcg P as plasmalogen choline, 5-13 mcg P as plasmalogen ethanolamine, 2-8 mcg P as plasmalogen serine, 1-3 mcg P as phosphatidic acid, 2-6 mcg P as diphosphoinositide and 5-13 mcg P as sphingomyelin.

Hereafter, for illustrative and not limitative purposes, examples 1-3 describe preparations made from infected bovine brains where the spongiform encephalopathy form was encountered or from protein raw materials obtained from uninfected bovine brains to which constant amounts of infected material from the 263K scrapie strain are added. Examples 4-18 illustrate pharmaceutical compositions with the purified phospholipids. The invention being thus described, it is clear that these methods can be modified in various ways. These modifications are not to be considered as deviations from the spirit and the purpose of the invention and all those modifications which would appear evident to one skilled in the art are comprised within the scope of the subsequent claims.

### EXAMPLE 1

1000 grams of infected frozen bovine brain, were ground to a fine powder. A small aliquot of powder was removed for the infectivity test and stored at -70°C.

All of the powder was dispersed in 900 ml of acetone for 15-30 minutes at room temperature. To this was added 1,1,1-trichloroethane to obtain a weight/volume ratio of 1:3. The suspension was left under vigorous magnetic stirring for 30 minutes at room temperature. It was centrifuged for 20 minutes at 1,000 rpm at 20°C and 500 ml of clear, lower, organic phase was recovered, discarding the upper, acetone/water phase and all insoluble material. It was subsequently left for 60 minutes in a thermostatic bath at a temperature of 7.5°C; it was then filtered through a Gooch funnel (pore size No. 3) to eliminate unsolubilized material. 1750 ml of acetone was added and the mixture was magnetically stirred for 30 minutes at a temperature of 15°C, and it was centrifuged for 10 minutes at 2000 rpm at 15°C. The precipitate (Raw Material 1) was repeatedly washed with 1000 ml of acetone and vacuum-dried at 15°C. 30 g of raw Material 1 was redissolved in 240 ml of a mixture of 1,1,1-trichloroethane and absolute ethanol in the ratio 5:2. Once solubilization was complete, 50 ml of distilled water was added and it was partitioned by shaking for 60 minutes at room temperature. It was then centrifuged at 1000 rpm until complete separation and clarification of the two phases, and the lower, organic phase was recovered. This operation was then repeated for a second time in the same conditions. A small aliquot was removed for biological assay and 720 ml of acetone was added to both these fractions. It was left for 30 minutes at a temperature of 15°C, then centrifuged again at 2000 rpm for 10 minutes. The precipitate was repeatedly washed with 500 ml of acetone. The product was vacuum-dried at 25°C (Raw Material 2). 2.4 g of raw Material 2 was resuspended in 300 ml of chloroform/methanol. To this was added 90 ml of an aqueous 0.4 molar solution of sodium chloride and it was partitioned by shaking for 30 minutes at room temperature. Finally the material was centrifuged at 1000 rpm till complete separation and clarification of the two phases, and the lower, organic phase was recovered. This operation was then repeated a second time in the same way. The final volume (180 ml) was divided into two identical aliquots to each of which was added the same amount of acetone, namely 540 ml. This was left to react for 30 minutes at a temperature of 15°C, and then centrifuged again at 2000 rpm. The precipitate was washed repeatedly with 500 ml of acetone. The product (10+10 g) was then vacuum-dried at 25°C and recovered in PBS (final product). An aliquot was sterilized at 121°C for 15 minutes then rapidly cooled at room temperature (final, sterilized product). Yield 2%.

The samples for biological assay were recovered in sterile PBS in the following volumes:

| | |
|---|---|
| Powdered, infected brain | ml 0.15 dilution 10⁰ |
| Intermediate product (Raw Material)2 | ml 0.33 dilution 10⁰ |
| Final product | ml 1.41 dilution 10¹ |
| Final, sterilized product | ml 1.41 dilution 10¹ |

(10⁰ means undiluted; 10¹ means diluted 10 times.)
Table 1 reports an example of the results obtained by assay of the biological activity.

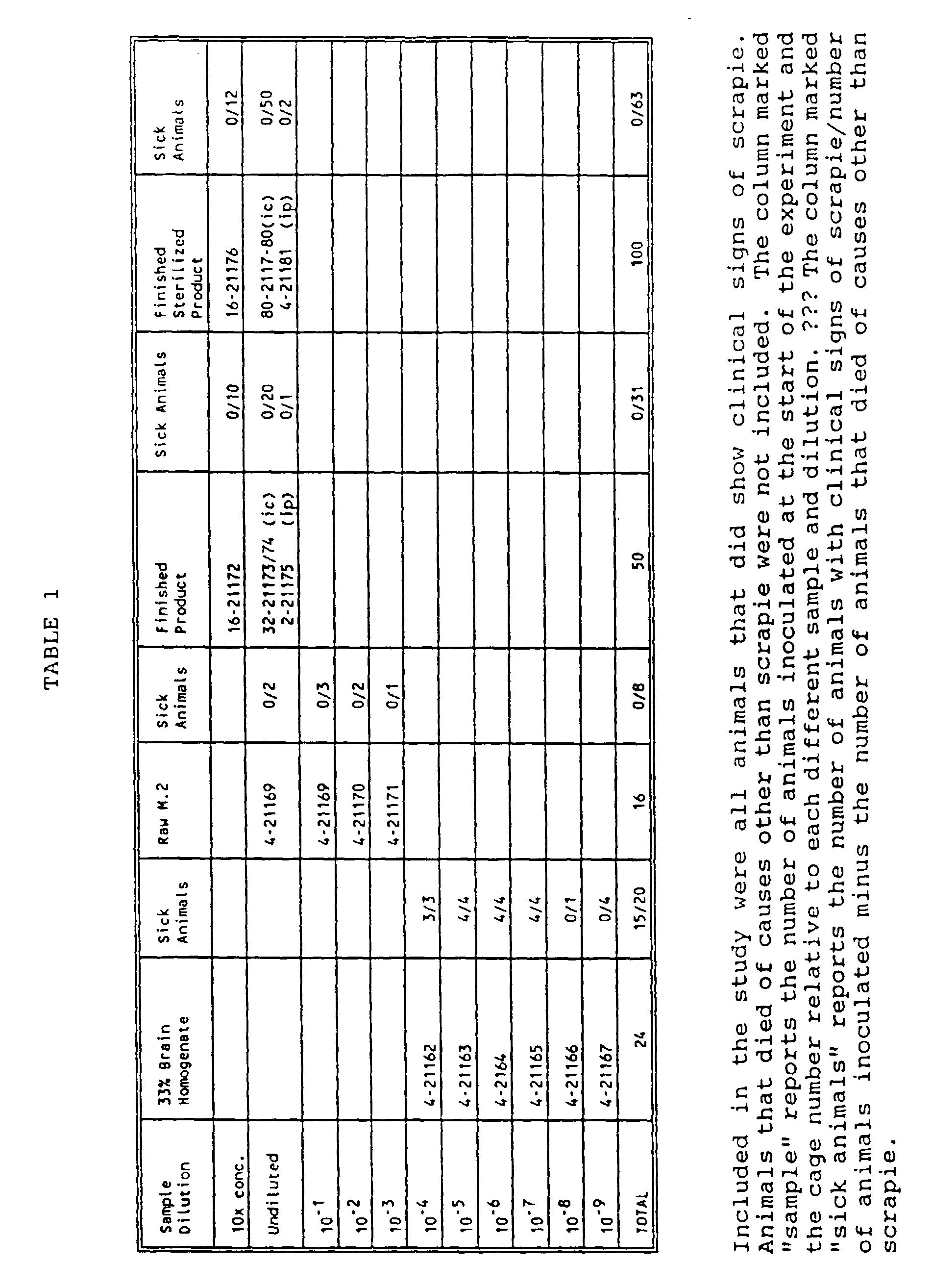

### EXAMPLE 2

1000 g of infected frozen bovine brain were ground to a fine powder. A small aliquot was then removed and stored at -70°C for the infectivity assay.

All of the powder was dispersed in 900 ml of acetone for 15-30 minutes at room temperature. To it was added ml of 1,1,1-trichloroethane to obtain a weight/volume ratio of 1:3. The suspension was left under vigorous magnetic stirring for 30 minutes at room temperature. It was centrifuged for 20 minutes at 1000 x rpm at 20°C and the clear, lower, organic phase was recovered (500 ml), discarding the upper, acetone/water phase and all insoluble material. It was then left for 60 minutes, in a thermostatic bath at a temperature of 7.5°C; it was then filtered through a Gooch funnel, pore size 3, to eliminate the insoluble material. 1750 ml of acetone was added and it was left under magnetic stirring for about 30 minutes at a temperature of 15°C, and it was centrifuged for 10 minutes at 2000 x rpm at +15°C. The precipitate (First Raw Material 1) was repeatedly washed with 1000 ml of acetone and left to vacuum-dry at 15°C. 30 g of raw Material 1 was redissolved in 240 ml of a mixture of 1,1,1-trichloroethane/absolute ethanol in the ratio 5:2. Once solubilization was complete, 50 ml of distilled water was added and it was partitioned by shaking for 60 minutes at room temperature. It was then centrifuged at 1000 rpm until complete separation and clarification of the two phases, and the lower, organic phase was recovered. This operation was repeated a second time in the same way. A small aliquot was removed for biological assay and 720 ml acetone was added to both of these fractions. It was left for 30 minutes at a temperature of 15°C, then centrifuged again at 2000 rpm for 10 minutes. The precipitate was washed repeatedly with 500 ml of acetone. The product was vacuum-dried at 25°C (Second Raw Material 2). The precipitate was repeatedly washed with acetone and resuspended in 300 ml of chloroform/methanol. The material was then loaded onto a silica gel column (6-35 µ) equilibrated in chloroform. The column was eluted with chloroform, chloroform/ethanol in the ratio 7:3, chloroform/methanol/water (65:25:4) and chloroform/methanol/water (50:50:12.5) (12.5 parts of water per volume) in turn. Each fraction was recovered for analysis by TLC chromatography (V.P. Skipski et al., Methods Enzymol., 14, 1969: 530; F, Vitiello et al., J. Chromatogr., 166, 1978: 637). After analysis the fractions eluted with chloroform/methanol/water (65:25:4) (4 parts of water per volume), corresponding to fractions 12-18, were recovered and vacuum-dried. They were then resuspended by sonication in PBS.

The samples for biological assay were recovered in sterile PBS in the following volumes:

| | |
|---|---|
| Final product | ml 0.8 dilution 10¹ |
| Final, sterilized product | ml 0.8 dilution 10¹ |

Table 2 reports an example of the results obtained by assay of the biological activity.

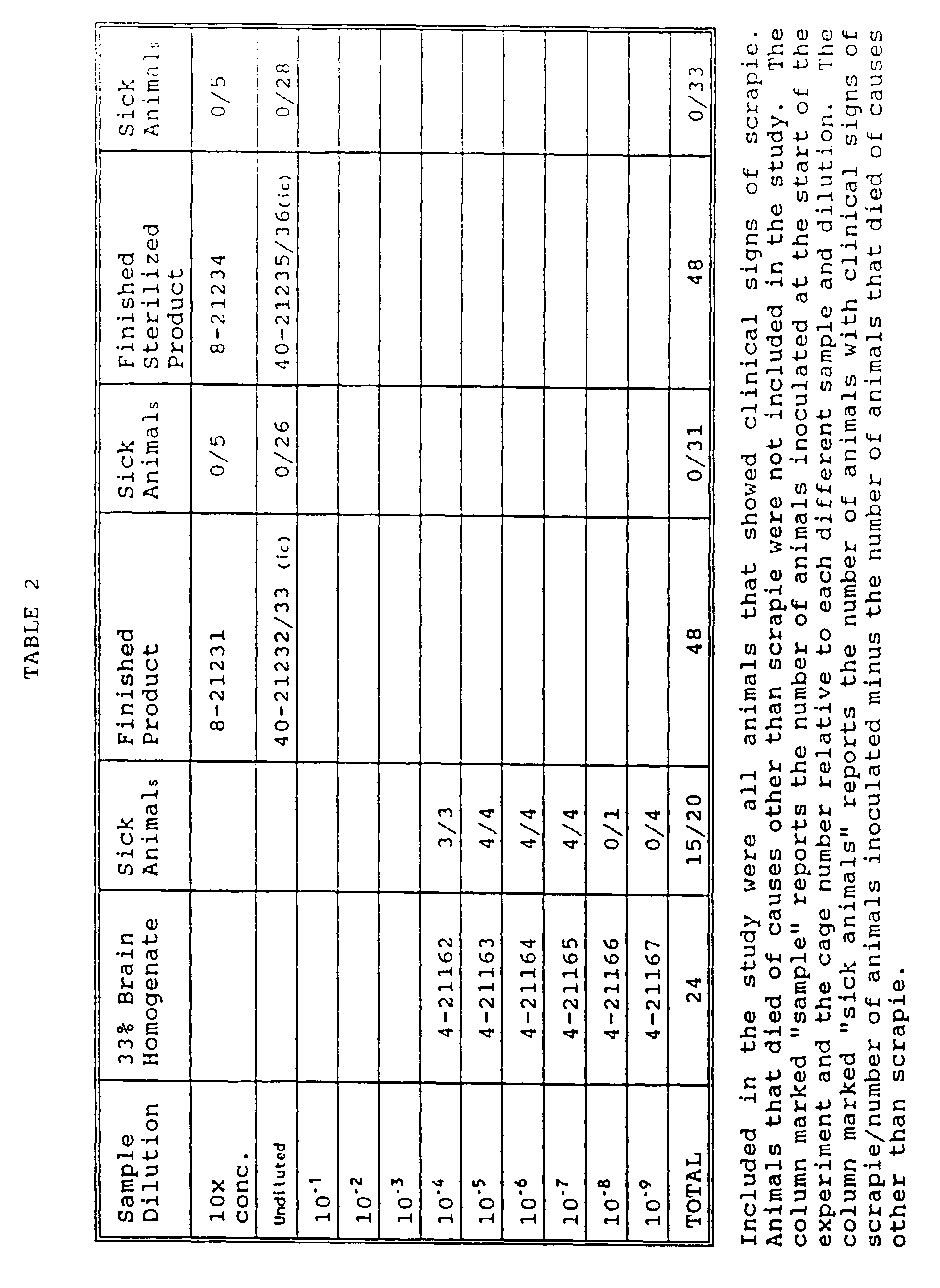

### EXAMPLE 3

5 g of silica gel (6-35 µ) were resuspended in a volume of 10-20 ml of chloroform for 5-15 minutes. The gel was loaded onto a vacuum-packed chromatographic column. The column was then washed thoroughly with chloroform. 50 mg of uninfected bovine phospholipids (prepared as described in Example 1; corresponds to "Raw Material 2") were resuspended in 0.5-1.0 ml of chloroform/methanol and a 25% (w/v) homogenate of infected hamster brain 263K was added. This homogenate had a infectivity titer of 9.2 log LD50/ml (Di Martino et al, Arch. Virol., 124, 1992: 111). An identical amount of infected material was added in the same volume of PBS (infected homogenate).

The sample was well shaken to disperse the aqueous phase and 10 µl were taken for biological assay (starting material). The sample was then loaded into a column where it was eluted with the following solvents and their mixtures: chloroform, chloroform/ethanol, (7:3) chloroform/methanol/water, (65:25:4) and chloroform/methanol/water (50:50:12.5) (12.5 parts of water in the organic phase volume).

The rate of flow through the column was between 3 and 10 ml/min, preferably 6 ml min, and fractions of 2 ml each were taken.

The fractions were recovered as follows:
- Fraction 1-8: Column front, cholesterol esters, cerebrosides
- Fraction 9-11: Sulfatides and traces of phosphatidylcholine
- Fraction 12-18: phospholipids
- Fraction 19-22: Hydrophilic products and traces of sphingomyelin.

Each fraction was withdrawn for TLC chromatographic analysis (J.C. Touchstone et al., J. Liq. Chromatogr. 6: 179, 1983; F, Vitiello et al., J. Chromatogr., 166, 1978: 637). After analysis the fractions eluted with chloroform/ methanol/water (4 parts of water per volume), corresponding to fractions 12-18, were recovered and vacuum-dried. They were then resuspended by sonication in PBS.

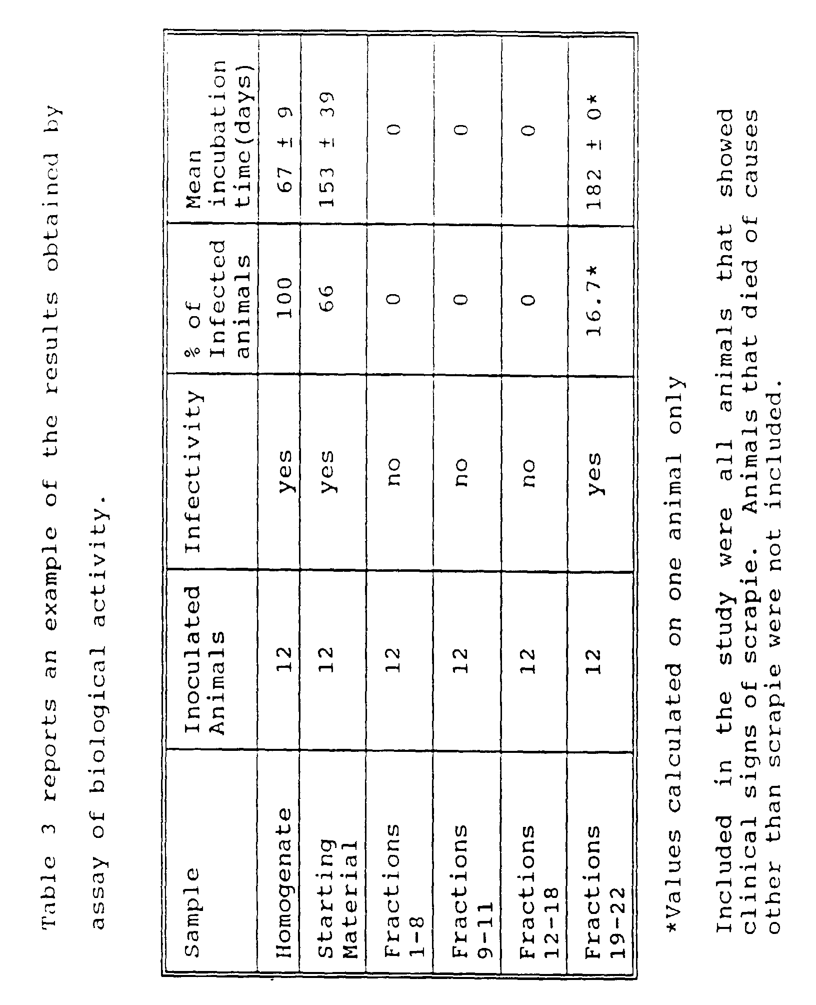

### EXAMPLE 4

One 2 ml ampoule is composed as follows: hypothalamic phospholipid liposomes corresponding to micrograms of lipidic phosphorous 400
- phosphatidylcholine 40%
- phosphatidylethanolamine + phosphatidylserine 34%
- sphingomyelin 10%
- other phospholipids present in small quantities (phosphatidylinositol, diphosphoinositide, phosphatidic acid, lysolecithin, lysocephalin) 16%

### Other components:

- lidocaine hydrochloride 2 mg
- esters of p-oxybenzoic acid 1.2 mg
- monobasic sodium phosphate 2H₂O 2.14 mg
- dibasic sodium phosphate 12 H₂O 2.26 mg
- water for injection q.s. ad 2 ml

### EXAMPLE 5

One 2-ml ampoule contains:
hypothalamic phospholipid liposomes corresponding to micrograms of lipidic phosphorous 1000
- phosphatidylcholine 40%
- phosphatidylethanolamine + phosphatidylserine 34%
- sphingomyelin 10%
- other phospholipids present in small quantities (phosphatidylinositol, diphosphoinositide, phosphatidic acid, lysolecithin, lysocephalin) 16%

### Other components:

- esters of p-oxybenzoic acid 1.2 mg
- monobasic sodium phosphate 2H₂O 2.14 mg
- dibasic sodium phosphate 12 H₂O 2.26 mg
- mannitol 100 mg
- water for injection q.s. ad 2 ml

### EXAMPLE 6

One 2-ml ampoule contains:
- phosphatidylcholine 140 mcg of P
- phosphatidylethanolamine 70 mcg of P
- phosphatidylserine 70 mcg of P
- plasmalogen-choline 4 mcg of P
- plasmalogen-ethanolamine 35 mcg of P
- plasmalogen-serine 20 mcg of P
- phosphatidic acid 8 mcg of P
- diphosphoinositide 15 mcg of P
- sphingomyelin 35 mcg of P
- Cyanocobalamin 1000 mcg
Other components:
- alpha-diethylamino-2-6 dimethylacetanilideHCl 2.4 mg
- esters of p-oxybenzoic acid 1.2 mg
- monobasic sodium phosphate 2H₂O 2.14 mg
- dibasic sodium phosphate 2H₂O 2.26 mg
- water for injection q.s. ad 2 ml

### EXAMPLE 7

One capsule contains:
- phospholipids from cortical grey matter 50 mg
constituted by:
- phosphatidylcholine 350 mcg of P
- phosphatidylethanolamine 175 mcg of P
- phosphatidylserine 175 mcg of P
- plasmalogen-choline 10 mcg of P
- plasmalogen-ethanolamine 85 mcg of P
- plasmalogen-serine 50 mcg of P
- phosphatidic acid 20 mcg of P
- diphosphoinositide 35 mcg of P
- sphingomyelin 85 mcg of P
- pyridoxine hydrochloride 150 mg
- cyanocobalamin 250 mcg
Other components:
- vegetable oil F.U. 200 mg
The outer shell is constituted by:
- gelatin F.U. 140 mg
- glycerin F.U. 45 mg
- sodium ethyl paraoxybenzoate 0.6 mg
- sodium propyl paraoxybenzoate 0.3 mg
- ethyl vanillin 0.5 mg
- titanium dioxide 2.1 mg
- erythrosine (E127) 2.6 mg

### EXAMPLE 8

One 2-ml ampoule contains:
- phosphatidylserine 50.00 mg
Other components:
- lecithin 15.00 mg
- mannitol 100.00 mg
- dibasic sodium phosphate-12H₂O 2.26 mg
- monobasic sodium phosphate-2H₂O 2.14 mg
- water for injection q.s. ad 2 ml

### EXAMPLE 9

One 2-ml ampoule contains:
- phosphatidylserine 50.00 mg
Other components:
- mannitol 100.00 mg
- dibasic sodium phosphate-12H₂O 2.26 mg
- monobasic sodium phosphate-2H₂O 2.14 mg
- water for injection q.s. ad 2 ml

### EXAMPLE 10

One 10-ml vial in which each ml contains:
- phosphatidylserine 25.00 mg
Other components:
- lecithin 7.50 mg
- mannitol 50.00 mg
- dibasic sodium phosphate-12H₂O 1.13 mg
- monobasic sodium phosphate-2H₂O 1.07 mg
- water for injection q.s. ad 1 ml

### EXAMPLE 11

One 10-ml vial in which each ml contains:
- phosphatidylserine 25.00 mg
Other components:
- mannitol 50.00 mg
- dibasic sodium phosphate-12H₂O 1.13 mg
- monobasic sodium phosphate-2H₂O 1.07 mg
- water for injection q.s. ad 1 ml

### EXAMPLE 12

One capsule contains:
- phosphatidylserine 100.00 mg
Other components:
- lecithin 30.00 mg
- vegetable oil 270.00 mg
The outer shell is constituted by:
- gelatin 129.00 mg
- glycerol 49.00 mg
- rust brown E 172 1.10 mg
- rust red E 172 0.40 mg
- sodium ethyl p-hydroxybenzoate 0.20 mg
- sodium propyl p-hydroxybenzoate 0.10 mg

### EXAMPLE 13

One capsule contains:
- phosphatidylserine 100.00 mg
Other components:
- vegetable oil 270.00 mg
The outer shell is constituted by:
- gelatin 129.00 mg
- glycerol 49.00 mg
- rust brown E 172 1.10 mg
- rust red E 172 0.40 mg
- sodium ethyl p-hydroxybenzoate 0.20 mg
- sodium propyl p-hydroxybenzoate 0.10 mg

### EXAMPLE 14

One vial complete with ampoule of solvent for parenteral administration, each vial containing:
Freeze-dried active component
- phosphatidylserine 50.00 mg
Other components:
- lecithin 15.00 mg
- mannitol 100.00 mg
- water for injection q.s. ad 4 ml

### EXAMPLE 15

One vial complete with ampoule of solvent for parenteral administration, each vial containing: Freeze-dried active component
- phosphatidylserine 50.00 mg
Other components:
- mannitol 100.00 mg
- water for injection q.s. ad 4 ml

### EXAMPLE 16

One single-dose pack (granules for oral use to mix with water before use) contains:
- phosphatidylserine 100.00 mg
Other components:
- sodium ascorbate 5.00 mg
- aspartame 5.00 mg
- colloidal silica 10.00 mg
- soybean lecithin 30.00 mg
- natural flavoring 40.00 mg
- mannitol 350.00 mg
- fructose q.s. ad 1.50 g

### EXAMPLE 17

One single-dose pack (granules for oral use to mix with water before use) contains:
- phosphatidylserine 100.00 mg
Other components:
- sodium ascorbate 5.00 mg
- aspartame 5.00 mg
- colloidal silica 10.00 mg
- natural flavoring 40.00 mg
- mannitol 350.00 mg
- fructose q.s. ad 1.50 g

### EXAMPLE 18

One single-dose pack (granules for oral use to mix with water before use) contains:
- phosphatidylserine 200.00 mg
Other components:
- sodium ascorbate 10.00 mg
- aspartame 20.00 mg
- colloidal silica 20.00 mg
- soybean lecithin 60.00 mg
- natural flavoring 80.00 mg
- mannitol 700.00 mg
- fructose q.s. ad 3.00 g

## Claims

1. A process for the preparation of a phospholipid mixture which is essentially free from infective components while maintaining the pharmacological properties of said phospholipid mixture comprising
a) extracting bovine brain with a mixture of acetone and a chlorinated hydrocarbon to obtain a crude extract comprising phospholipids,
b) filtering said crude extract to obtain a filtrate,
c) adding a precipitating agent to said filtrate to obtain a precipitate,
d) isolating said precipitate to obtain a first raw phospholipid-comprising mixture,
e) solubilizing the first raw mixture in a solvent mixture,
f) partitioning said first raw mixture with water and separating the phases,
g) precipitating the phospholipid material from the organic phase containing it and isolating the precipitate,
h) drying the precipitate to obtain a second phospholipid-comprising mixture
optionally
i) solubilizing said second phospholipid-comprising mixture in a solvent mixture,
j) partitioning said second mixture with saline and separating the phases,
k) precipitating the phospholipid material from the organic phase containing it and isolating the precipitate,
l) drying the precipitate to obtain a final phospholipid-comprising mixture
or
m) suspending said second mixture in a suspending liquid comprising a chlorinated hydrocarbon,
n) loading the suspension so formed on a silica gel column,
o) eluting said column with a mixture comprising organic and aqueous solvents, and
p) isolating the product after step h), step l), or step o).

2. The process according to Claim 1 wherein the solvent mixture used for extracting nervous tissue comprises 1,1,1-trichloroethane and acetone.

3. The process according to Claim 1 or 2 wherein the mixture of organic and aqueous solvents used for elution in step O) comprises a chlorinated hydrocarbon and at least one alcohol.

4. The process according to Claim 3 wherein said chlorinated hydrocarbon is chloroform.

5. The process according to Claim 3 wherein said alcohol is selected from the group consisting of methanol, ethanol, propanols, butanols and pentanols.

6. The process according to Claim 1 further comprising a step for recovering said phospholipid mixture from relevant eluted fractions in step O).

7. The process according to claim 6 wherein said recovery is performed by vacuum drying.

8. The process according to Claim 1 for the preparation of a phospholipid mixture completely free from unconventional viral agents while maintaining the pharmacological properties.

9. The process according to Claim 1 which is performed at room temperature for an extended period of time.

10. The process according to Claim 1 wherein the infective components are associated with unconventional agents.

11. The process according to Claim 1 wherein the infective components are "slow viruses".

12. The process according to Claim 1 wherein the infective components are associated with bovine spongiform encephalopathy.

## Patentansprüche

1. Verfahren zur Herstellung eines Phospholipidgemisches, das im wesentlichen frei ist von infektiösen Bestandteilen, während die pharmakologischen Eigenschaften des Phospholipidgemisches erhalten bleiben, umfassend
a) Extraktion von Rinderhirn mit einem Gemisch aus Aceton und einem chlorierten Kohlenwasserstoff zum Erhalt eines Phospholipide umfassenden Rohextrakts,
b) Filtration des Rohextrakts zum Erhalt eines Filtrats,
c) Zugabe eines Fällungsmittels zu dem Filtrat zum Erhalt eines Niederschlags,
d) Isolierung des Niederschlags zum Erhalt eines ersten Phospholipid umfassenden Rohgemisches,
e) Solubilisierung des ersten Rohgemisches in einem Lösungsmittelgemisch,
f) Ausschütteln des ersten Rohgemisches mit Wasser und Trennung der Phasen,
g) Ausfällen des Phospholipidmaterials aus der es enthaltenden organischen Phase und Isolierung des Niederschlags,
h) Trocknen des Niederschlags zum Erhalt eines zweiten Phospholipid enthaltenden Gemisches,
gegebenenfalls
i) Solubilisieren des zweiten Phospholipid enthaltenden Gemisches in einem Lösungsmittelgemisch,
j) Ausschütteln des zweiten Gemisches mit physiologischer Kochsalzlösung und Trennung der Phasen,
k) Ausfällen des Phospholipidmaterials aus der es enthaltenden organischen Phase und Isolierung des Niederschlags,
l) Trocknen des Niederschlags zum Erhalt eines letzten Phospholipid umfassenden Gemisches,
oder
m) Suspendieren des zweiten Gemisches in einer einen chlorierten Kohlenwasserstoff umfassenden Suspensionsflüssigkeit,
n) Auftragen der so erhaltenen Suspension auf eine Kieselgelsäule,
o) Elution der Säule mit einem Gemisch aus organischen und wäßrigen Lösungsmitteln, und
p) Isolieren des nach Schritt h), Schritt 1) oder Schritt o) erhaltenen Produkts.

2. Verfahren nach Anspruch 1, wobei das für die Extraktion von Nervengewebe verwendete Lösungsmittelgemisch 1,1,1-Trichlorethan und Aceton umfaßt.

3. Verfahren nach Anspruch 1 oder 2, wobei das für die Elution in Schritt o) verwendete Gemisch aus organischen und wäßrigen Lösungsmitteln einen chlorierten Kohlenwasserstoff und mindestens einen Alkohol umfaßt.

4. Verfahren nach Anspruch 3, wobei der chlorierte Kohlenwasserstoff Chloroform ist.

5. Verfahren nach Anspruch 3, wobei der Alkohol ausgewählt ist aus Methanol, Ethanol, Propanolen, Butanolen und Pentanolen.

6. Verfahren nach Anspruch 1, weiterhin umfassend einen Schritt zur Gewinnung des Phospholipidgemischs aus den entsprechenden eluierten Fraktionen in Schritt o).

7. Verfahren nach Anspruch 6, wobei die Gewinnung durch Vakuumtrocknen erfolgt.

8. Verfahren nach Anspruch 1 für die Herstellung eines Phospholipidgemisches, das vollständig frei ist von ungewöhnlichen ("unconventional") viralen Agentien, wobei die pharmakologischen Eigenschaften erhalten bleiben.

9. Verfahren nach Anspruch 1, das für einen längeren Zeitraum bei Raumtemperatur durchgeführt wird.

10. Verfahren nach Anspruch 1, wobei die infektiösen Bestandteile mit ungewöhnlichen Agentien assoziiert sind.

11. Verfahren nach Anspruch 1, wobei die infektiösen Bestandteile "langsame Viren" sind.

12. Verfahren nach Anspruch 1, wobei die infektiösen Bestandteile mit boviner spongiformer Encephalopathie (BSE) assoziiert sind.

## Revendications

1. Un procédé de préparation d'un mélange de phospholipide qui est pratiquement exempt de constituants infectants tout en conservant les propriétés pharmacologiques dudit mélange de phospholipides, comprenant :
a) l'extraction de cerveaux bovins à l'aide d'un mélange d'acétone et d'hydrocarbure chloré pour obtenir un extrait brut comprenant les phospholipides,
b) la filtration dudit extrait brut pour obtenir un filtrat,
c) l'addition d'un agent de précipitation audit filtrat pour obtenir un précipité,
d) l'isolement dudit précipité pour obtenir un premier mélange brut renfermant les phospholipides,
e) la solubilisation du premier mélange brut dans un mélange de solvants,
f) le partage dudit premier mélange brut à l'aide d'eau et séparation des phases,
g) la précipitation de la substance phospholipidique de la phase organique la renfermant et l'isolement du précipité,
h) le séchage du précipité pour obtenir un second mélange comprenant le phospholipide,
éventuellement,
i) la solubilisation dudit mélange comprenant le phospholipide dans un mélange de solvants.
j) le partage dudit second mélange à l'aide d'eau salée et séparation des phases,
k) la précipitation de la substance phospholipidique de la phase organique la renfermant et l'isolement du précipité,
l) le séchage du précipité pour obtenir un mélange final comprenant le phospholipide,
ou bien
m) la mise en suspension dudit second mélange dans un liquide de suspension comprenant un hydrocarbure chloré,
n) le chargement de la suspension ainsi préparée sur une colonne de silica gel,
o) l'élution de ladite colonne à l'aide d'un mélange comprenant des solvants organiques et aqueux,
p) l'isolement du produit après l'étape h, l'étape l ou l'étape o.

2. Le procédé selon la revendication 1 dans lequel le mélange, de solvants utilisé pour l'extraction du tissu nerveux comprend du 1,1,1-trichloroethane et de l'acétone.

3. Le procédé selon la revendication 1 ou 2 dans lequel le mélange de solvants organiques et aqueux utilisé pour l'élution dans l'étape o comprend un hydrocarbure chloré et au moins un alcool.

4. Le procédé selon la revendication 3 dans lequel ledit hydrocarbure chloré est le chloroforme.

5. Le procédé selon la revendication 3 dans lequel ledit alcool est choisi dans le groupe consistant en méthanol, éthanol, propanols, butanols et pentanols.

6. Le procédé selon la revendication 1 comprenant en outre une étape de récupération dudit mélange phospholipidique à partir des fractions éluées appropriées de l'étape o.

7. Le procédé selon la revendication 6 dans lequel ladite récupération est effectuée par séchage sous vide.

8. Le procédé selon la revendication 1 de préparation d'un mélange phospholipidique complètement débarrassé d'agents viraux non classiques tout en conservant les propriétés pharmacologiques.

9. Le procédé selon la revendication 1 mis en oeuvre à la température ambiante pendant une durée prolongée.

10. Le procédé selon la revendication 1 dans lequel les composants infectieux sont associés à des agents non classiques.

11. Le procédé selon la revendication 1 dans lequel les composants infectieux sont des "virus lents".

12. Le procédé selon la revendication 1 dans lequel les composants infectieux sont associés à l'encéphalopathie spongiforme bovine.
